# FASCICULE DE BREVET EUROPEEN

(11) **EP 3 562 325 B1**
(45) Date de publication et mention de la délivrance du brevet: **01.10.2025**
(21) Numéro de dépôt: 17832246.7
(22) Date de dépôt: 28.12.2017
(51) Int. Cl.: A01K 5/02, A23K 10/18, A23K 10/28, A23K 10/33, A23K 10/37, A23K 20/163, A23K 20/174, A23K 20/20, A01K 67/30, A23P 30/00, A23N 17/00, A23K 10/38, A23K 50/90, B01J 13/00, A23L 29/20

(54) **PROCEDE D'APPORT EN EAU DANS L'ELEVAGE D'INSECTES**
VERFAHREN ZUR WASSERVERSORGUNG IN DER INSEKTENAUFZUCHT
PROCESS FOR WATER SUPPLY IN RAISING INSECTS

(30) Priorité: 29.12.2016 FR 1663522
(43) Date de publication de la demande: 06.11.2019
(73) Titulaire: YNSECT, 91058 Évry-Courcouronnes Cedex (FR); CARGILL, Incorporated, Wayzata, MI 55391 (US)
(72) Inventeur: COMPARAT, Solene, 91000 Evry (FR); CLESSE, Loïc, 80240 Roisel (FR); DU JONCHAY, Thibault, 60710 Chevrières (FR); LEFEBVRE, Thomas, 91450 Soisy Sur Seine (FR); BERRO, Fabrice, 75001 Paris (FR); BEREZINA, Nathalie, 75005 Paris (FR); SEGUIMBRAUD, Clémentine, 50500 Baupte (FR)
(74) Mandataire: Santarelli
(86) Numéro de dépôt international: PCT/EP2017/084784
(87) Numéro de publication internationale: WO 2018/122361

(56) Documents cités:
- WO-A1-2016/004312
- WO-A2-02/26048
- FR-A1- 2 157 819
- US-A- 5 643 622
- US-A1- 2009 285 937
- US-B1- 6 293 223
- MOADELI TAHEREH ET AL: "High productivity gel diets for rearing of Queensland fruit fly,Bactrocera tryoni", JOURNAL OF PEST SCIENCE, SPRINGER BERLIN HEIDELBERG, BERLIN/HEIDELBERG, vol. 90, no. 2, 17 November 2016 (2016-11-17), pages 507 - 520, XP036164420, ISSN: 1612-4758, [retrieved on 20161117], DOI: 10.1007/S10340-016-0813-0

## Description

La présente invention concerne un procédé d'apport en eau dans un élevage d'insectes.

Elle s'applique aux élevages d'insectes.

Les insectes visés par l'invention sont par exemple les coléoptères, les diptères, les lépidoptères, les isoptères, les orthoptères, les hyménoptères, les blattoptères, les hémiptères, les hétéroptères, les éphéméroptères et les mécoptères, de préférence, les coléoptères, les diptères, les orthoptères, les lépidoptères.

Le terme insecte est employé pour désigner tout stade d'évolution de l'œuf ou oothèque à l'insecte adulte, et l'invention vise plus particulièrement l'élevage des insectes du stade larvaire à l'insecte adulte.

L'élevage d'insectes requiert un apport en eau et en éléments nutritifs nécessaires à la survie des insectes ainsi qu'à leur croissance et leur bon développement. Il est connu d'apporter tout ou partie de la nourriture et de l'eau sous forme d'un composé aqueux gélifié.

Par exemple, le document US 6 293 223 divulgue un milieu nutritif gélifié pour l'élevage de larves.

Dans les exemples connus, des éléments nutritifs, par exemple solides, sont mélangés avec de l'eau et un gélifiant tel que de l'agar à une température adaptée. Le composé obtenu est gélifié en le ramenant à plus faible température.

Le composé gélifié ainsi obtenu est ensuite débité en blocs de taille adaptée. Les blocs sont conditionnés pour être expédiés vers un élevage de larves.

Selon un autre mode de réalisation connu, le composé liquide est versé dans un bac comportant des alvéoles de taille correspondant aux blocs souhaités, où il est refroidi et gélifié avant démoulage des alvéoles du bac.

Un tel composé aqueux gélifié constitue une source de nourriture et d'eau facile d'emploi, qui ne nécessite pas de structure de distribution particulière dans les contenants ou cages d'élevage, et permettant un apport d'eau en limitant les risques de noyade des insectes. Néanmoins, si l'emploi d'un composé gélifié présente de nombreux avantages dans le cadre d'un élevage d'insectes, il présente des inconvénients ou des risques liés notamment au transport, à la manutention, et au stockage du composé qui est susceptible dans chacune de ces opérations d'être contaminé, ou de développer des moisissures.

US 2009/0285937 divulgue un supplément alimentaire pour insectes ainsi qu'une méthode de production d'un pastille de gel aqueux pour insectes. Pour retarder la croissance bactérienne durant le stockage, un enrobage imperméable est proposé.

L'invention vise à proposer un procédé permettant de résoudre au moins l'un des inconvénients précités.

Ainsi, l'invention porte sur un procédé d'apport en eau dans un élevage d'insectes, comportant :
- une étape de formation d'un composé par mélange :
   i. d'un substrat aqueux, liquide à température ambiante, porté à une température permettant la dissolution d'un gélifiant ; et
   ii. du gélifiant,
- une étape de soutirage du composé ;
- une étape de refroidissement en ligne du composé de sorte à l'amener en dessous d'une deuxième température, à laquelle il est gélifié ;
- une étape de transfert dans une ligne de distribution ;
- une étape de débitage en blocs du composé gélifié, en sortie de ligne de distribution ; et
- une étape de distribution dans un contenant d'élevage d'insectes des blocs de gel à une température compatible pour le nourrissage et l'apport en eau à des insectes, immédiatement successive au débitage en bloc du gel.

En gélifiant le composé en ligne, après soutirage sous forme liquide, et en le débitant en blocs directement en sortie d'une ligne de distribution, le gel est produit au besoin et en continu. La manutention du gel et son stockage (sous forme de gel) sont éliminés, ce qui supprime de fait les problématiques associées. Les risques de contamination ou de développement de bactéries sont fortement limités, car le gel est distribué immédiatement en sortie de ligne de distribution (qui est essentiellement close), peu de temps après que le composé a été formé à une température élevée. Par ailleurs, dans le cadre d'un élevage d'insectes, la taille des blocs en sortie peut être adaptée aux besoins de manière fine, et en continu.

Dans un mode de réalisation de l'invention, le procédé comporte, avant l'étape de soutirage du composé, le refroidissement dudit composé et son maintien dans une plage de température, inférieure à la température permettant la dissolution du gélifiant mais suffisante pour maintenir le composé à l'état liquide, ladite plage de température permettant l'ajout sans dégradation de compléments susceptibles d'être dégradés à ladite température permettant la dispersion du gélifiant.

Le refroidissement comporte par exemple l'ajout d'eau ou de substrat aqueux à température ambiante ou à une température inférieure à la température ambiante, afin d'amener le composé dans la plage de température, et le maintien en température du composé comporte la régulation de la température par des activations et des arrêts d'un moyen de chauffage du composé.

Le moyen de chauffage employé peut être du type à vapeur circulant dans une double paroi d'une cuve employée pour l'étape de dissolution et pour l'étape de maintien en température, l'activation du moyen de chauffage comportant l'envoi de vapeur dans la double paroi.

Par exemple, la plage de température dans laquelle est maintenu le composé peut être définie de sorte à maintenir le composé à une viscosité inférieure à 10 000 cPo. La plage de température pour l'étape de maintien en température peut être définie par deux bornes respectivement choisies entre 45°C et ladite température permettant la dissolution du gélifiant.

Dans une variante possible du procédé, le composé est à l'état de gel en dessous de 40°C.

La température permettant la dissolution d'un gélifiant à laquelle le substrat aqueux est porté peut être comprise entre 60°C et 100°C, notamment entre 60°C et 95°C, par exemple de l'ordre de 95°C ou de l'ordre de 75°C.

Le gélifiant employé peut comporter un ou plusieurs éléments choisis parmi : l'agar-agar, le carraghénane, la gomme de guar, l'alginate de calcium, le chitosan, la pectine, la gomme de xanthane, la gomme de caroube, la gomme de gellane.

Le procédé peut également comporter l'ajout d'au moins un complément parmi des vitamines, un probiotique, un agent conservateur, des minéraux.

Le substrat aqueux employé peut être de l'eau ou peut comporter un coproduit liquide d'agro-industrie et présenter une teneur en eau supérieure à 35% en poids, notamment une teneur en eau supérieure à 50% en poids, sur le poids total de gel.

Le substrat aqueux peut comporter au moins l'un des coproduits de l'industrie agricole ou agro-alimentaire suivants :
- un soluble de maïs, de blé, de pois, de manioc, de betterave à sucre, de canne à sucre ;
- un solubles de distillerie, notamment de distillerie de blé, de maïs, de pois, du manioc ;
- une vinasse ;
- une mélasse ;
- une crème de levure ;
- du lactosérum.

On décrit également ici un dispositif d'apport en eau dans un élevage d'insectes, comportant un dispositif de production d'un gel aqueux en blocs pour apporter de l'eau aux insectes comportant :
- une cuve comportant un ou plusieurs disperseurs ;
- une arrivée d'un substrat aqueux liquide à température ambiante, débouchant dans la cuve ;
- des moyens de contrôle et de pilotage de la température dans la cuve ;
- des moyens de soutirage d'un contenu liquide de la cuve ;
- un système de dosage;
- un échangeur de refroidissement du contenu liquide soutiré de la cuve ;
- au moins une ligne de distribution en sortie de l'échangeur ;
- un distributeur de gel disposé en bout de chaque ligne de distribution et comportant un dispositif de débitage adapté à sectionner un gel en blocs ; et
- des moyens pour amener un contenant d'élevage d'insectes en dessous du distributeur.

Dans un tel dispositif, le système de dosage peut comporter une pompe volumétrique, par exemple une pompe doseuse à piston. Le dispositif peut par exemple comporter une pompe volumétrique pour chaque ligne de distribution.

Le dispositif de distribution peut comporter une un système de découpe automatisé (par exemple une électrovanne) adapté à sectionner le gel en sortie de ligne de distribution.

La ligne de distribution et le dispositif de débitage peuvent être configurés pour la réalisation de blocs de gel d'un volume compris entre 30 et 1500 cm³.

Les moyens de contrôle et de pilotage de la température dans la cuve peuvent comporter un capteur de température dans la cuve.

La cuve peut comporter une double paroi dotée d'une arrivée de vapeur entre les deux parois et une vanne pilotée sur l'arrivée de vapeur.

Le dispositif peut en outre comporter :
- une première arrivée de substrat aqueux à une première température comportant une vanne pilotée et un débitmètre; et
- une deuxième arrivée de substrat aqueux à une deuxième température comportant une vanne pilotée et un débitmètre.

D'autres particularités et avantages de l'invention apparaîtront encore dans la description ci-après.

Aux dessins annexés, donnés à titre d'exemples non limitatifs :
- la figure 1 représente schématiquement par un logigramme un procédé conforme à un mode de réalisation de l'invention ;
- la figure 2 représente schématiquement un dispositif permettant la mise en œuvre d'un procédé conforme à un mode de réalisation de l'invention.

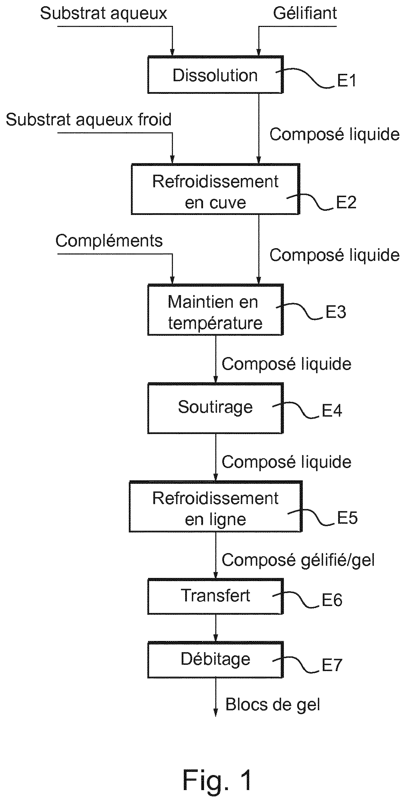

La figure 1 représente un logigramme détaillant la succession des étapes mises en œuvre dans la réalisation des blocs de gel. Le mode de réalisation représenté comporte les étapes essentielles ainsi qu'un certain nombre d'étapes propres au mode de réalisation représenté.

Un substrat aqueux, c'est-à-dire un produit contenant de l'eau, liquide à température ambiante, est fourni. La notion de température ambiante désigne une température classiquement rencontrée dans un atelier de préparation en l'absence de chauffage ou de refroidissement du milieu. La « température ambiante » peut notamment désigner une température comprise entre 5°C et 35°C. La notion de liquide comprend des produits fluides présentant une viscosité typiquement jusque 10 000 cPo.

Le substrat aqueux peut être de l'eau. Le substrat aqueux employé peut être un liquide contenant au moins 35% d'eau en poids, et de préférence entre 55% et 98,2%. Par exemple, le substrat aqueux employé peut être un liquide contenant entre 60% et 95%, et préférentiellement entre 70% et 90% d'eau en poids sur le poids total de substrat aqueux.

Notamment, de nombreux coproduits d'agro-industrie peuvent être employés. Le substrat aqueux peut par exemple être constitué d'un mélange d'eau et d'un coproduit de l'agro-industrie. Par exemple, le substrat aqueux peut être constitué d'eau et d'au moins 25% en poids, par exemple au moins 50% en poids, par exemple 75% en poids, de coproduit de l'agro-industrie.

Le gel produit peut typiquement comporter :
- de 90% à 99,6% en poids d'un substrat aqueux comportant au moins 25% en poids sur le poids total de substrat aqueux d'un coproduit liquide de l'agro-industrie,
- de 0,3 à 2% en poids d'un agent gélifiant, et
- de 0,1 à 5% en poids d'un agent conservateur,
les pourcentages en poids de substrat aqueux, d'agent gélifiant et d'agent conservateur étant exprimés sur le poids total du gel.

Un coproduit est une matière inévitable créée au cours d'un processus de fabrication d'un produit d'intérêt.

En particulier, le coproduit visé par l'invention est liquide. Par « liquide », on entend que le coproduit est sous forme liquide à température ambiante dans les conditions normales de pression atmosphérique. En particulier, cela signifie que c'est un coproduit obtenu directement à l'issu d'un procédé industriel sans qu'aucune étape de séchage n'ait été réalisée.

Plus particulièrement, le coproduit liquide est un coproduit aqueux comportant des matières solubles. Préférentiellement, les matières solubles présentes dans le coproduit liquide sont des protéines et/ou des glucides tels que du saccharose et/ou du lactose, plus préférentiellement, des protéines et des glucides. Les matières solubles peuvent également comporter des fibres solubles.

Avantageusement, le coproduit liquide comporte au moins 90% en poids de matières solubles sur le poids total de matière sèche.

Par agro-industrie, on vise plus particulièrement les industries de l'amidonnerie, de la féculerie, de la malterie, de production de bioéthanol, du sucre, de la fermentation, de la brasserie, de la distillation et l'industrie laitière.

Plus particulièrement, le coproduit est un coproduit aqueux comportant des matières solubles. Préférentiellement, les matières solubles présentes dans le coproduit sont des protéines et/ou des glucides tels que du saccharose et/ou du lactose, plus préférentiellement, des protéines et des glucides. Les matières solubles peuvent également comporter des fibres solubles.

Le coproduit peut également être ou comporter une crème de levure, typiquement résultant d'un procédé de fabrication de bioéthanol, ou plus généralement des industries de la fermentation. Les crèmes de levures correspondent aux coproduits résultant de séparation d'un moût telle que par filtration ou par centrifugation après fermentation. Les industries de la fermentation produisent, en tant que coproduit utilisable, également des vinasses, qui sont des coproduits liquides issus de la fermentation du moût après extraction des composés d'intérêts.

L'industrie sucrière génère plusieurs sortes de coproduits liquides pouvant être employés, et notamment les égouts de sucrerie et la mélasse. Les égouts de sucrerie et la mélasse correspondent aux résidus sirupeux obtenus après cristallisation de la liqueur formée durant la fabrication du sucre.

Il en résulte que le coproduit liquide peut être choisi en particulier parmi la liste comprenant : les solubles de céréales, les solubles de maïs, les solubles de blé, les solubles de pois, les solubles de manioc, les solubles de betterave à sucre, les solubles de canne à sucre, les solubles de distillerie de céréales, les solubles de distillerie de blé, les solubles de distillerie de maïs, les solubles de distillerie de pois, les solubles de distillerie du manioc, les vinasses, les mélasses, les crèmes de levures, les lactosérums et leurs dérivés concentrés notamment le perméat, ou leurs mélanges.

Le gel peut comporter des levures. Les levures peuvent provenir du coproduit liquide de l'agro-industrie. Le coproduit de l'agro-industrie peut en effet être un soluble de distillerie qui comporte déjà des levures ou un mélange d'au moins deux coproduits liquides de l'agro-industrie dont l'un est une crème de levures.

Alternativement, les levures peuvent être ajoutées sous forme solide, par exemple, sous forme de levures sèches ou comme indiquées ci-après de probiotique. Sous forme de levures sèches, elles sont introduites à une teneur comprise entre 0,1 à 6% en poids, préférentiellement, entre 1 et 5% en poids sur le poids total du gel.

Le substrat aqueux est porté à une température souhaitée, en vue de la dissolution dans le substrat aqueux d'un gélifiant. La température souhaitée peut typiquement être comprise entre 60°C et 100°C, notamment de l'ordre de 95°C, ou de l'ordre de 75°C, selon le gélifiant employé. Le substrat aqueux est fourni à cette température avant introduction dans une cuve, ou amené à cette température une fois dans ladite cuve.

Dans une étape de dissolution E1, un gélifiant est ajouté au substrat aqueux. Le gélifiant peut être, ou peut comporter, par exemple : l'agar-agar, le carraghénane, la gomme de guar, l'alginate de calcium, le chitosan, la pectine, la gomme de xanthane, la gomme de caroube, la gomme de gellane ou leurs mélanges.

Du fait de la température suffisante du substrat aqueux, le gélifiant est dissout dans le substrat aqueux. A la température (qui reste quasiment invariante car la quantité de gélifiant ajoutée dans le substrat est faible en proportion dudit substrat) le composé ainsi formé est liquide.

Dans l'étape de dissolution E1, un mélange est opéré afin d'obtenir un composé liquide dans lequel le gélifiant est réparti de manière homogène. Le mélange est opéré dans une enceinte adaptée, typiquement une cuve.

Un agent conservateur peut également être ajouté, par exemple à une teneur comprise entre 0,1% et 5% en poids du gel obtenu en fin de procédé, préférentiellement entre 0,15% et 0,5%, par exemple 0,3%. L'agent conservateur peut être choisi parmi le groupe constitué par l'acide acétique, l'acétate de sodium, l'acide formique, le lactate de sodium, l'acide fumarique, l'acide sorbique, l'acide propionique, l'acide citrique, le sorbate de potassium, le sorbate de calcium, le propionate de sodium, la propionate de calcium, le benzoate de sodium, l'acide benzoïque, le benzoate de calcium, le benzoate de potassium, l'acide butyrique, ainsi que les sels et acides correspondant à ces molécules.

De préférence, le conservateur n'est pas un paraben.

L'exemple de procédé ici représenté comporte une étape de refroidissement E2 en cuve lors de laquelle le composé est amené à une température, inférieure à la température de dissolution du gélifiant, mais supérieure à la température de gélification du composé. Le refroidissement peut être obtenu par ajout d'une quantité de substrat aqueux froid permettant l'atteinte de la température souhaitée. Un substrat aqueux froid correspond au substrat aqueux à température ambiante ou à une température inférieure. Le substrat aqueux froid est préférentiellement à l'état liquide. Le substrat aqueux ajouté est préférentiellement le même, c'est-à-dire de même composition, que le substrat déjà présent dans le composé. Il peut s'agir d'eau.

Le mélange du composé en vue de garantir son homogénéité (dans sa composition que dans sa température) est poursuivi tout au long de l'étape de refroidissement E2 en cuve.

La quantité de substrat aqueux froid ajoutée pour le refroidissement en cuve est pré-évaluée ou préétablie, de sorte qu'elle est prise en compte lors de l'ajout de gélifiant pour l'étape de dissolution E1, de sorte que le composé contienne une proportion de gélifiant située dans une plage prédéterminée, après l'étape de refroidissement E2.

Typiquement, le composé final (après ajout d'éventuels compléments comme détaillée à l'étape E3 décrite ci-après) peut comporter entre 0,3% et 2% en poids de gélifiant. Par exemple, un gel à base d'eau gélifiée par un gélifiant de xanthane-caroube à parts égales, peut avantageusement comporter entre 0,3% et 0,8% en poids de gélifiant.

La teneur en gélifiant du composé va influencer la force du gel obtenu au final. Aussi, dans le cadre d'un élevage d'insectes, et selon le stade de développement des insectes auxquels est destiné le gel, un gel ayant une force d'au moins 30g/cm², notamment comprise entre 30g/cm² et 150g/cm², par exemple de l'ordre de 50 g/cm² ou de l'ordre de 80g/cm² (à température ambiante, par exemple à 20°C) peut être avantageusement employé. Ainsi, le gel n'est pas gluant ou collant. Les insectes peuvent donc se déplacer au-dessus du gel sans être englués. Cela réduit donc la mortalité des insectes, les insectes se retrouvant moins prisonniers du gel. En outre, la synérèse du gel peut être avantageusement comprise entre 0,1 et 5% afin d'éviter un relargage d'eau trop important et de mouiller l'environnement des insectes. La synérèse du gel peut être déterminée, par exemple, comme indiqué dans G. BLANCHER (2009), Sciences du Vivant, ENSIA (AgroParisTech). La mesure est effectuée sur des produits conservés à 4°C pendant 24 h, par pesée différentielle avec une balance analytique. Brièvement, le produit contenu dans un godet est pesé, puis le liquide contenu en surface est enlevé en inclinant le godet puis avec un papier absorbant légèrement appuyé sur la surface du produit. Une deuxième pesée est ensuite effectuée. La synérèse est exprimée en % de perte entre les deux pesées.

Après l'étape de refroidissement E2, le composé liquide est maintenu à une température de consigne, ou dans une plage de température souhaitée, dans une étape de maintien en température E3.

Cette régulation en température peut être réalisée par activation (i.e. mise en fonctionnement) de moyens de chauffage lorsque le composé se refroidit et pourrait sortir de la plage de température de régulation, et arrêt desdits moyens de chauffage lorsque le composé se réchauffe et pourrait sortir de la plage de température de régulation.

Le mélange du composé en vue de garantir son homogénéité (tant quant à sa composition qu'à sa température) est poursuivi tout au long de l'étape de maintien en température E3.

La plage de température de régulation est choisie de sorte à maintenir le composé dans un état liquide, par exemple à une viscosité inférieure à 10 000 cPo. La plage de température est également choisie, le cas échéant, pour permettre l'introduction dans le composé de compléments pouvant être dégradés par une température excessive, sans dégrader lesdits compléments. Les compléments ajoutés lors de l'étape de maintien en température E3 sont par exemple des vitamines, un probiotique, un agent conservateur, ou un mélange de tels compléments, ou tout autre composé thermosensible ayant un intérêt pour la formulation nutritionnelle. Par exemple, un probiotique peut être ajouté à une teneur comprise entre 0,1% et 8% en poids (par exemple entre 1% et 5%) du gel obtenu en fin de procédé, et/ou des vitamines ajoutées sous la forme d'un « prémix » (pouvant contenir également des minéraux et des oligoéléments) avec une teneur en « prémix » comprise entre 0,1 % et 5% en poids du gel obtenu en fin de procédé. Les minéraux et/ou les oligoéléments peuvent alternativement être ajoutés indépendamment d'un prémix ou en complément d'un prémix. Notamment, les vitamines ajoutées peuvent être choisies parmi la vitamine A, la vitamine B1 (thiamine), la vitamine B2 (riboflavine), la vitamine B3 (nicotinamide), la vitamine B5 (acide pantothénique), la vitamine B6 (pyridoxine), la vitamine B8 (biotine), la vitamine B9 (acide folique), la vitamine B12 (cobalamine), la vitamine PP (Niacine), la vitamine D3 (cholécalciférol), la Vitamine E, la vitamine K3 (ménadione), leurs précurseurs, leurs dérivés.

Par exemple, une plage de température comprise entre 45°C et 65°C est généralement adaptée. Toute plage comprise dans ces bornes, par exemple une plage de 50°C à 60°C peut être envisagée. Plus la plage est étroite et plus la régulation en température doit être fine.

Le composé liquide à la température de maintien (ou régulation) est ensuite soutiré de la cuve (ou autre enceinte) dans laquelle il a été formé, et est réparti et pompé à l'aide d'une ou plusieurs pompe doseuses dans une ou plusieurs lignes, dans une étape de soutirage E4 et de dosage.

Le composé liquide est ensuite gélifié par refroidissement dans une étape de refroidissement en ligne E5. L'étape de refroidissement en ligne amène le composé à une température inférieure à sa température de gélification, qui peut être par exemple de l'ordre de 40°C. Plus généralement, le composé ainsi gélifié est amené à une température compatible de l'usage pour lequel il est prévu. Par exemple, pour le nourrissage et l'apport en eau à des insectes, le composé, qui sera distribué à une température proche de sa température après refroidissement en ligne E5 est amené à une température maximale de 25°C en sortie du refroidissement en ligne E5.

Le refroidissement en ligne E5 peut être réalisé en une fois, ou via plusieurs paliers de refroidissement, par des refroidissements graduels et successifs.

Le gel ainsi obtenu est transféré, dans une étape de transfert E6, vers une ligne de distribution du gel. La ligne de distribution mène le gel vers son point d'utilisation. Pour la distribution de gel pour le nourrissage ou l'apport d'eau à des insectes, la ligne de distribution débouche dans ou au-dessus de contenants d'élevage, qui sont successivement amenés à la sortie de la ligne de distribution.

Au niveau de la sortie ou peu avant la sortie d'une ligne de distribution, le gel convoyé dans la ligne de distribution est sectionné afin d'être débité en blocs, dans une étape de débitage E7. Le gel est ainsi distribué sous forme de blocs de gel. Le gel distribué est employé immédiatement.

Le volume des blocs dépend de sa destination. Dans le cadre d'un élevage d'insectes, des blocs de gel ayant un volume compris entre 30 cm3 et 1500 cm3 peuvent par exemple être produits. Les blocs peuvent avoir la forme parallélépipède (par exemple un cube, ou un parallélépipède à base carrée), ou un cylindre, dont la longueur est de l'ordre de 0,5 à 15 cm, préférentiellement de 0,8 à 12 cm.

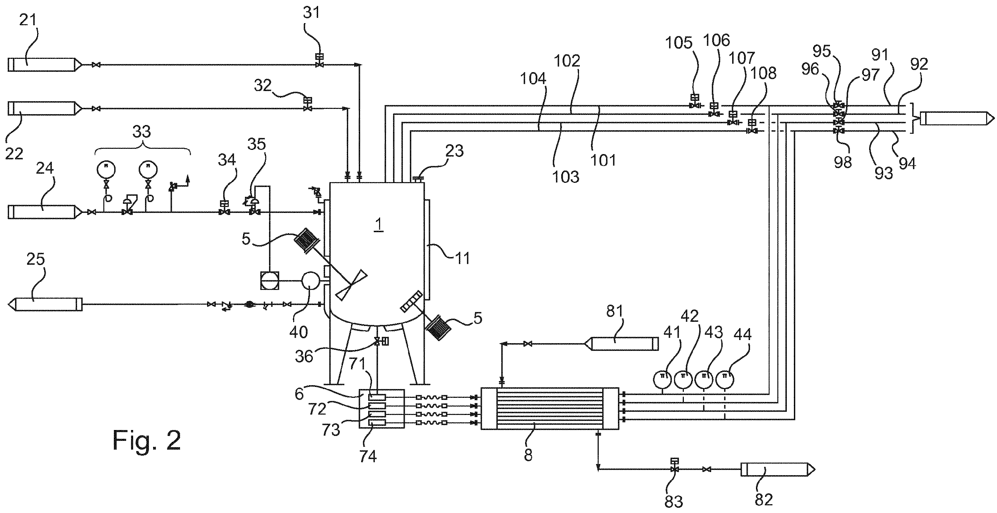

La figure 2 présente de manière schématique un dispositif industriel permettant la mise en œuvre du procédé décrit ci-dessus.

Le dispositif comporte une cuve 1.

La cuve 1 est alimentée par :
- une arrivée de substrat aqueux froid 21, par exemple d'eau, à température ambiante ou inférieure. L'arrivée de substrat aqueux froid 21 est dotée d'une vanne d'arrivée de substrat aqueux froid 31, qui peut être une vanne pilotée ; et
- une arrivée de substrat aqueux chaud 22, par exemple d'eau. L'arrivée de substrat aqueux chaud 22 est dotée d'une vanne d'arrivée de substrat aqueux froid 32, qui peut être une vanne pilotée. Le substrat aqueux chaud est à une température permettant la dissolution d'un gélifiant, par exemple de l'ordre de 75°C.

La cuve comporte en outre une entrée 23 permettant l'introduction dans la cuve 1 de produits supplémentaires. L'entrée 23 peut être employée par exemple pour l'ajout d'un gélifiant ou de compléments.

La cuve 1 est du type à double paroi, ménageant un espace 11 entre les parois de la cuve 1. Une arrivée de vapeur 24 débouche dans l'espace 11. L'arrivée de vapeur 24 est dotée d'un certain nombre de dispositifs de régulation 33, notamment de régulation de la pression de la vapeur. L'arrivée de vapeur 24 est dotée d'une vanne d'arrivée vapeur 34, qui peut être une vanne pilotée. L'arrivée de vapeur 24 est également dotée d'une vanne pilotée 35, dont l'ouverture est pilotée en fonction de la température du composé dans la cuve. A cette fin, et le cas échéant pour le contrôle d'autres fonctions, un capteur de température dans la cuve 40 permet la mesure de la température du composé dans la cuve. Plusieurs capteurs de température dans la cuve peuvent être prévus et répartis spatialement dans la cuve, pour éviter les dispersions de mesures et s'assurer en outre de la bonne homogénéité de la température du composé dans la cuve.

Le contrôle de la vanne pilotée 35 permet ainsi par exemple la régulation en température du composé présent dans la cuve, c'est-à-dire son maintien à une température de consigne ou dans une plage de température prédéfinie.

L'arrivée de vapeur 24 est avantageusement située en haut de cuve.

La cuve 1 est également dotée d'une sortie des condensats 25 issus de la vapeur refroidie et condensée suite au transfert de chaleur à la paroi interne de la cuve 1 et au composé qu'elle contient.

Dans un mode de réalisation non représenté, la double enveloppe de la cuve 1 peut être configurée pour recevoir, dans le même espace que la vapeur ou dans un volume dédié, de l'eau froide permettant le refroidissement du contenu de la cuve 1.

La cuve 1 est dotée d'au moins un disperseur 5. Dans l'exemple ici représenté, la cuve 1 est dotée de deux disperseurs 5. Les disperseurs 5 permettent le mélange du composé présent dans la cuve 1, et la dispersion rapide et homogène de tout produit, liquide ou pulvérulent, ajouté dans le composé.

Une vanne de soutirage 36, située sur une ligne de sortie en fond de la cuve 1, permet le soutirage du composé présent dans la cuve 1. Une trémie 6 est configurée pour répartir le composé soutiré en plusieurs lignes. Bien évidemment, le nombre de lignes dépend de la variante de l'invention considérée, et l'invention se rapporte également à un dispositif ne comportant qu'une seule ligne.

Chaque ligne est dotée d'une pompe volumétrique, ou pompe doseuse. La pompe doseuse employée doit permettre le pompage dans la ligne du composé d'abord sous forme liquide, puis sous forme de gel. Une pompe à piston est particulièrement bien adaptée pour cela.

Dans l'exemple ici représenté, le dispositif comporte quatre lignes après la trémie 6 : une première ligne est équipée d'une première pompe doseuse 71, une deuxième ligne est équipée d'une deuxième pompe doseuse 72, une troisième ligne est équipée d'une troisième pompe doseuse 73, et une quatrième ligne est équipée d'une quatrième pompe doseuse 74.

Le composé, encore liquide dans chacune des lignes, traverse alors un échangeur 8.

L'échangeur 8 est du type liquide/liquide. Il refroidit le composé en faisant circuler un liquide froid, typiquement de l'eau, autour d'un faisceau de tubes dans lequel le composé est acheminé. L'échangeur 8 comporte une entrée d'eau 81 de refroidissement et une sortie d'eau 82.

L'échangeur 8 permet d'amener le composé sous sa température de gélification. Ainsi, le composé sort de l'échangeur 8 sous la forme d'un gel, à la température souhaitée pour sa distribution (ou proche de la température souhaitée). Une vanne de sortie d'eau de refroidissement 83 peut permettre d'arrêter l'écoulement d'eau dans l'échangeur 8. Dans une variante de l'invention, la vanne de sortie d'eau 83 peut être une vanne pilotée, permettant une régulation de la température du gel en sortie de l'échangeur 8 en pilotant le débit de l'eau traversant l'échangeur 8.

L'eau de refroidissement peut circuler en boucle fermée et être refroidie avant d'être ramenée à l'entrée d'eau 81.

Dans des variantes non représentées de l'invention, l'échangeur 8 peut être remplacé par une succession d'échangeurs en série. En outre, chaque ligne pourrait être équipée de son propre échangeur.

Afin de contrôler la température du gel en sortie de l'échangeur 8, chaque ligne est équipée d'un capteur de température dans la ligne. Le dispositif comporte ainsi un capteur de température dans la première ligne 41, un capteur de température dans la deuxième ligne 42, un capteur de température dans la troisième ligne 43, et un capteur de température dans la quatrième ligne 44.

Le composé sous forme de gel est ensuite transféré dans une ligne de distribution, ou dans une ligne de retour vers la cuve. Le dispositif ici représenté comporte quatre lignes de distribution : une première ligne de distribution 91, une seconde ligne de distribution 92, une troisième ligne de distribution 93 et une quatrième ligne de distribution 94. Le dispositif ici représenté comporte quatre lignes de retour dans la cuve correspondante : une première ligne de retour 101, une deuxième ligne de retour 102, une troisième ligne de retour 103 et une quatrième ligne de retour 104.

Chaque ligne de distribution est équipée d'un distributeur de gel disposé en bout ligne. Le distributeur de gel comporte en particulier un dispositif de débitage adapté à sectionner un gel en blocs. Le dispositif de débitage peut comporter une vanne, notamment de type « stop-goutte », permettant de sectionner le gel de manière nette. Le dispositif de débitage peut en particulier être un système de découpe automatisé. Le système de découpe automatisé peut être typiquement pneumatique ou électrique. Par exemple, la première ligne de distribution 91 comporte une première électrovanne 95, la deuxième ligne de distribution 92 comporte une deuxième électrovanne 96, la troisième vanne de distribution 93 comporte une troisième électrovanne 97 et la quatrième ligne de distribution 94 comporte une quatrième électrovanne 98. En sortie de ligne de distribution, le distributeur fourni ainsi des blocs de gels, de volume fixe ou variable, le volume étant déterminé par la longueur de gel débité par une ligne avant section.

Les lignes de retour permettent un retour du composé dans la cuve 1. Un tel retour peut être nécessaire lors de l'amorçage du soutirage. En effet, lors de l'amorçage cette circulation en boucle fermée peut permettre d'éliminer l'air présent dans les lignes du dispositif. En outre, lors de l'amorçage le gel peut présenter une température trop élevée en sortie de l'échangeur 8. Un retour de composé dans la cuve 1 peut également être nécessaire lorsque, pour quelque raison que ce soit, la quantité de composé dosée dans une ligne excède la quantité à distribuer. Un retour de composé dans la cuve 1 peut également être nécessaire lorsqu'une circulation du composé est souhaitée dans une ligne, alors que sa distribution n'est pas souhaitée. Cela peut par exemple être le cas si on souhaite une distribution de gel à une température donnée, supérieure à la température ambiante, et qu'un trop long séjour dans les lignes du composé l'amènerait à une température trop basse pour sa distribution. Enfin, le retour vers la cuve peut être employé, en dehors des phases de production de gel, pour le nettoyage du dispositif. Le retour peut être régulé par, respectivement : une première vanne de retour 105, une deuxième vanne de retour 106, une troisième vanne de retour 107, et une quatrième vanne de retour 108.

Dans le cadre d'un élevage d'insectes, le dispositif est complété par des moyens pour amener un contenant d'élevage d'insectes en dessous du distributeur. Ainsi, le distributeur permet de déposer un ou plusieurs blocs de gel, de volume prédéfini ou adapté pour chaque contenant, directement après productions dans le contenant, sans manipulation supplémentaire du gel.

L'invention propose ainsi la formation d'un gel d'un substrat aqueux, par exemple d'un gel d'eau, produit *in situ* et distribué sous forme de blocs à la demande, en continu. La manutention du gel et son stockage sont éliminés, ce qui supprime de fait les problématiques associées, notamment de contamination ou de pourrissement. Par ailleurs, dans le cadre d'un élevage d'insectes, la taille des blocs en sortie peut être adaptée aux besoins, et en continu.

## Revendications

1. Procédé d'apport en eau dans un élevage d'insectes, comportant :
- une étape de formation d'un composé par mélange :
i. d'un substrat aqueux, liquide à température ambiante, porté à une température permettant la dissolution (E1) d'un gélifiant ; et
ii. du gélifiant,
- une étape de soutirage (E4) du composé ;
- une étape de refroidissement en ligne (E5) du composé de sorte à l'amener en dessous d'une deuxième température, à laquelle il est gélifié ;
- une étape de transfert (E6) dans une ligne de distribution ;
- une étape de débitage (E7) en blocs du composé gélifié, en sortie de ligne de distribution ; et
- une étape de distribution dans un contenant d'élevage d'insectes des blocs de gel à une température compatible pour le nourrissage et l'apport en eau à des insectes, immédiatement successive au débitage en bloc du gel.

2. Procédé selon la revendication 1, comportant, avant l'étape de soutirage (E4) du composé, le refroidissement (E2) dudit composé et son maintien dans une plage de température (E3), inférieure à la température permettant la dissolution du gélifiant mais suffisante pour maintenir le composé à l'état liquide, ladite plage de température permettant l'ajout sans dégradation de compléments susceptibles d'être dégradés à ladite température permettant la dispersion du gélifiant.

3. Procédé selon la revendication 2, dans lequel le refroidissement comporte l'ajout d'eau ou de substrat aqueux à température ambiante ou à une température inférieure à la température ambiante, afin d'amener le composé dans la plage de température, et le maintien en température du composé comporte la régulation de la température par des activations et des arrêts d'un moyen de chauffage du composé.

4. Procédé selon à la revendication 2 ou la revendication 3, dans lequel la plage de température pour l'étape de maintien en température (E3) est définie par deux bornes respectivement choisies entre 45°C et ladite température permettant la dissolution du gélifiant.

5. Procédé selon l'une quelconque des revendications précédentes, dans lequel la température permettant la dissolution d'un gélifiant à laquelle le substrat aqueux est porté est comprise entre 60°C et 100°C.

6. Procédé selon l'une des revendications précédentes, dans lequel le gélifiant comporte un ou plusieurs éléments choisis parmi : l'agar-agar, le carraghénane, la gomme de guar, l'alginate de calcium, le chitosan, la pectine, la gomme de xanthane, la gomme de caroube, la gomme de gellane.

7. Procédé selon l'une des revendications précédentes, comportant l'ajout d'au moins un complément parmi des vitamines, un probiotique, un agent conservateur, des minéraux.

8. Procédé selon l'une des revendications précédentes, dans lequel le substrat aqueux est de l'eau, ou comporte un coproduit liquide d'agro-industrie et présente une teneur en eau supérieure à 35% en poids.

9. Procédé selon l'une des revendications précédentes, dans lequel le substrat aqueux comporte au moins l'un des coproduits de l'industrie agricole ou agro-alimentaire suivants :
- un soluble de maïs, de blé, de pois, de manioc, de betterave à sucre, de canne à sucre ;
- un solubles de distillerie, notamment de distillerie de blé, de maïs, de pois, du manioc ;
- une vinasse ;
- une mélasse ;
- une crème de levure ;
- du lactosérum.

## Patentansprüche

1. Verfahren zur Wasserzufuhr in einer Insektenzucht, das Folgendes aufweist:
- einen Schritt des Bildens einer Verbindung durch Mischen:
i. eines wässrigen, flüssigen Substrats bei Raumtemperatur, das auf eine Temperatur gebracht wird, die das Auflösen (E1) eines Geliermittels ermöglicht; und
ii. des Geliermittels,
- einen Schritt des Entnehmens (E4) der Verbindung;
- einen Schritt zum Abkühlen der Verbindung in eine Linie (E5), um sie unter eine zweite Temperatur zu bringen, bei der sie geliert;
- einen Schritt zum Übertragen (E6) in eine Verteilerlinie;
- einen Schritt (E7) des Zuschneidens der gelierten Verbindung in Blöcke am Ausgang der Verteilerlinie; und
- einen Schritt des Verteilens der Gelblöcke in einem Behälter zur Insektenzucht bei einer geeigneten Temperatur für das Füttern und Zuführen von Wasser an Insekten, unmittelbar nach dem Zuschneiden des Gels in Blöcke.

2. Verfahren nach Anspruch **1,** das vor dem Schritt des Entnehmens (E4) der Verbindung das Abkühlen (E2) der Verbindung und deren Aufrechterhaltung in einem Temperaturbereich (E3) aufweist, der niedriger ist als die Temperatur, die das Auflösen des Geliermittels ermöglicht, aber ausreichend ist, um die Verbindung in flüssigem Zustand zu halten, wobei der Temperaturbereich das Hinzufügen, ohne Abbau, von Ergänzungsmitteln ermöglicht, die bei dieser Temperatur abgebaut werden können und die Dispersion des Geliermittels ermöglichen.

3. Verfahren nach Anspruch 2, wobei das Kühlen das Hinzufügen von Wasser oder wässrigem Substrat bei Raumtemperatur oder bei einer Temperatur unter der Raumtemperatur aufweist, um die Verbindung in den Temperaturbereich zu bringen, und das Aufrechterhalten der Temperatur der Verbindung die Temperaturregelung durch Aktivieren und Stoppen eines Heizmittels der Verbindung aufweist.

4. Verfahren nach Anspruch 2 oder Anspruch 3, wobei der Temperaturbereich für das Aufrechterhalten der Temperatur (E3) durch zwei Klemmen definiert ist, die jeweils zwischen 45 °C und der Temperatur ausgewählt sind, die das Auflösen des Geliermittels ermöglicht.

5. Verfahren nach einem der vorhergehenden Ansprüche, wobei die Temperatur, die das Auflösen des Bildens eines Geliermittels ermöglicht, auf das das wässrige Substrat gebracht wird, zwischen 60 °C und 100 °C liegt.

6. Verfahren nach einem der vorhergehenden Ansprüche, wobei das Geliermittel eines oder mehrere Elemente aufweist, ausgewählt aus: Agar-Agar, Carrageen, Guargummi, Calciumalginat, Chitosan, Pektin, Xanthangummi, Johannisbrotkernmehl, Gellangummi.

7. Verfahren nach einem der vorhergehenden Ansprüche, das das Hinzufügen von mindestens einem Ergänzungsmittel unter Vitaminen, einem Probiotikum, einem Konservierungsmittel, Mineralien aufweist.

8. Verfahren nach einem der vorhergehenden Ansprüche, wobei das wässrige Substrat Wasser ist oder ein flüssiges Nebenprodukt der Agrarindustrie aufweist und einen Wassergehalt von mehr als 35Gew.-% enthält.

9. Verfahren nach einem der vorhergehenden Ansprüche, wobei das wässrige Substrat mindestens eines der folgenden Nebenprodukte der Agrar- oder Lebensmittelindustrie aufweist:
- eine Lösung von Mais, Weizen, Erbsen, Cassava, Zuckerrüben, Zuckerrohr;
- ein Lösungsmittel aus Brennereien, insbesondere aus Brennereien für Weizen, Mais, Erbsen, Cassava;
- eine Vinasse;
- Melasse;
- Hefecreme
- Molke.

## Claims

1. Method for providing water in an insect farm, comprising:
- a step of forming a compound by mixing:
i. an aqueous substrate, liquid at room temperature, brought to a temperature allowing the dissolution (E1) of a gelling agent; and
ii. the gelling agent,
- a step of dispensing (E4) the compound;
- a step of in-line cooling (E5) of the compound so as to bring it below a second temperature, at which it is gelled;
- a step of transfer (E6) in a distribution line;
- a step of dispensing (E7) the gelled compound into blocks, at the outlet of the distribution line; and
- a step of dispensing the gel blocks, into an insect breeding container, at a compatible temperature for feeding and providing water to insects, immediately following block dispensing of the gel.

2. Method according to claim 1, comprising, before the step of dispensing (E4) the compound, cooling (E2) said compound and maintaining it in a temperature range (E3), lower than the temperature allowing the dissolution of the gelling agent but sufficient to maintain the compound in the liquid state, said temperature range allowing the addition without degradation of supplements likely to be degraded at said temperature allowing the dispersion of the gelling agent.

3. Method according to claim 2, wherein cooling comprises adding water or aqueous substrate at room temperature or at a temperature below room temperature, in order to bring the compound into the temperature range, and maintaining the temperature of the compound comprises regulating the temperature by activating and stopping a means of heating the compound.

4. Method according to claim 2 or claim 3, wherein the temperature range for the temperature maintenance step (E3) is defined by two bounds respectively selected between 45°C and said temperature allowing the gelling agent to dissolve.

5. Method according to any one of the preceding claims, wherein the temperature allowing the dissolution of a gelling agent to which the aqueous substrate is brought is between 60°C and 100°C.

6. Method according to one of the preceding claims, wherein the gelling agent comprises one or more elements selected from: agar-agar, carrageenan, guar gum, calcium alginate, chitosan, pectin, xanthan gum, carob gum, gellan gum.

7. Method according to one of the preceding claims, comprising the addition of at least one supplement among vitamins, a probiotic, a preservative agent, minerals.

8. Method according to one of the preceding claims, wherein the aqueous substrate is water, or includes a liquid agro-industry coproduct and has a water content greater than 35% by weight.

9. Method according to one of the preceding claims, wherein the aqueous substrate includes at least one of the following coproducts of the agricultural or agro-food industry:
- a soluble of maize, wheat, peas, cassava, sugar beet, sugar cane;
- distillery solubles, in particular wheat, maize, peas or cassava distillery;
- a vinasse;
- molasses;
- cream of yeast;
- whey.
